# EUROPEAN PATENT APPLICATION

(11) **EP 2 862 942 A1**
(43) Date of publication of application: **22.04.2015**
(21) Application number: 13382411.0
(22) Date of filing: 17.10.2013
(51) Int. Cl.: C12Q 1/68

(54) **Detection of Streptococcus pneumoniae**

(71) Applicant: Genomica S.A.U., 28823 Coslada, Madrid (ES)
(72) Inventor: Villahermosa Jaen, María Luisa, 28823 Coslada, Madrid (ES); Moraga Quintanilla, Ana Isabel, 28823 Coslada, Madrid (ES)
(74) Representative: Fleck, Barbara

(57) **Abstract**

The present invention is based on a detection method of *Streptococcus pneumoniae* in a sample, which allows specifically distinguish *Streptococcus pneumoniae* from other species of the *Streptococcus* genus.

Further, the present invention relates to (i) a kit which comprises a microarray wherein probes of the present invention are provided; (ii) the set of probes itself; (iii) a microarray comprising the set of probes; and (iv) use of the method, kit, set of probes, and microarray of above for detection of *Streptococcus pneumoniae* in a sample.

## Description

### Background of the invention.

*Streptococcus pneumoniae* is a causative agent of sepsis, and it would be convenient to be able to specifically detect its presence in blood. *Streptococcus pneumoniae* is also a major cause of community-acquired pneumonia. It is of great importance to be able to distinguish *Streptococcus pneumoniae* from the other members of the *Streptococci* group, in order to determine whether any *Streptococcus* present in a sample corresponds actually to pathogenic *Streptococcus pneumoniae* species, or to other innocuous *Streptococcus* species constitutively present in the host, and which do not cause disease.

Many attempts have been carried out in order to detect *Streptococcus pneumoniae* and distinguish it from the other members of the *Streptococci* group (Wessels et al., J. Clin. Microbiol. April 2012 vol. 50 no. 4 1171-1177). These include culture testing and conventional biochemical and phenotypic tests, such as Gram stain morphology, optochin susceptibility, colony morphology, and alpha-hemolysis on sheep blood agar. Further techniques that are used include: MALDI-TOF (MS matrix-assisted laser desorption ionization-time of flight mass spectrometry), sequence analysis of several genes, and molecular assays including Real-Time PCR assay. Most of these techniques, however, do not allow discriminate *Streptococcus pneumoniae* out of other members of the *Streptococci* group, due to the high sequence similarity between the different species. Other techniques include molecular methods based on amplification of the region of the gene rpoB for detection of bacteria of the genus *Streptococcus.* Two particular examples of this approach are provided in EP1558637 and EP1694861. EP1558637 makes use of a 724 bp fragment of the rpoB gene (displayed as SEQ ID Nº 12 in WO2004/041841), for detection of bacteria of the *Streptococcus* genus that may be present in a sample. Such detection may take place through amplification of the indicated fragment. No particular probe is, however, provided for the specific detection of different species of the *Streptococcus* genus.
In EP1694861 a fragment of the rpoB gene is amplified with a pair of primers that is common to all the species of the *Streptococcus* genus. An amplification product is thereby obtained, irrespective of the particular species that may be present in the sample, as in EP1558637 above. In the present case probes are provided, which are intended for the specific detection of *Streptococcus pneumoniae* and *Streptococcus pyogenes* (Table 3 of corresponding WO 2005/059156). The minimal sequence differences between some of the probes, however, may prove difficult that such probes may actually be useful for a specific detection of the two different *Streptococcus* species they target (e.g.: probes of SEQ ID Nº 4 and SEQ ID Nº 5, corresponding to *Streptococcus pyogenes* and *Streptococcus pneumoniae,* respectively, just differ in two nucleotides). No additional probes corresponding to other species of the *Streptococcus* genus are provided.

In view of the information above, it seems that the state of the art does not anticipate effective methods based on probe hybridization for the specific detection of *Streptococcus pneumoniae vs* other species of the *Streptococcus* genus. It would thus be highly desirable to provide for a method that may allow specifically detect *Streptococcus pneumoniae* present in a sample, and distinguish it from other species of the *Streptococcus* genus.

### Summary of the invention.

The problem to be solved by the present invention is, thereby, the provision of a method for detection of *Streptococcus pneumoniae* present in a sample, which allows specifically distinguish *Streptococcus pneumoniae* from other species of the *Streptococcus* genus.

The solution is based on the simultaneous use of probes of SEQ ID Nº 1 and SEQ ID Nº 2 (see Table 1 below), to detect the presence of *Streptococcus pneumoniae* in a sample.

As indicated above, there is a high sequence similarity between the different species of the *Streptococcus* genus; this circumnstance constitutes a heavy burden when trying to detect *Streptococcus pneumoniae* and specifically distinguish it from other *Streptococcus* species, through probe hybridization. The probes of SEQ ID Nº 1 and SEQ ID Nº 2 allow efficiently distinguish *Streptococcus pneumoniae* from, at least, the following species of the *Streptococcus* genus: *Streptococcus (S.) constellatus, S. anginosus, S. mitis, S. gallolyticus, S. dysgalactiae, S. pyogenes, S*. *suis, S*. *intermedius, S. sanguinis, S. oralis and S. parasanguinis.*

It is mandatory that simultaneous detection through hybridization with the two probes of SEQ ID Nº 1 and SEQ ID Nº 2 is carried out, for the specific detection of *Streptococcus pneumoniae.* Only when DNA present in a sample corresponds to *Streptococcus pneumoniae,* binding to both these probes takes place. In cases where none or only one of the probes of SEQ ID Nº 1 and SEQ ID Nº 2 binds DNA present in a sample, this DNA proves not to correspond to *Streptococcus pneumoniae,* but to other species of the *Streptococcus* genus instead. The only exception is the case of a sample containing DNA of *Streptococcus mitis,* in which case sample DNA binds to probe of SEQ ID Nº 1, binding of DNA to probe of SEQ ID Nº 2 also taking place to a certain level, but to a much lower extent than to probe of SEQ ID Nº 1 (see Example 2 below). As much lower extent it is understood a binding level of 20% or lower than the one achieved with SEQ ID Nº 1.

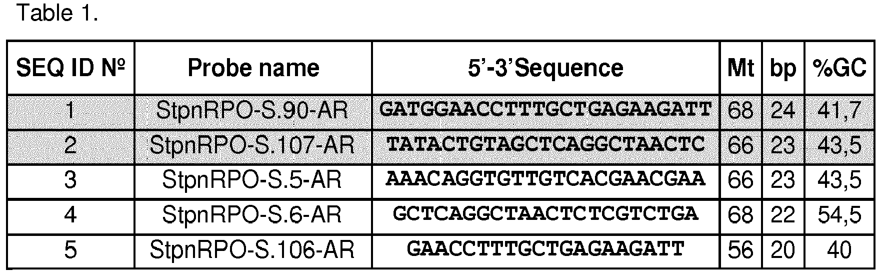

Probes of SEQ ID Nº 3, SEQ ID Nº 4 and SEQ ID Nº 5 of Table 1, also bind nucleic acids of *Streptococcus pneumoniae.* However, they have proven to be non-specific, as they also arbitrarily bind nucleic acids of other species of the *Streptococcus* genus, without following any kind of predictable pattern.

There is not any pointer in the state of the art nor in the probes sequences to explain why simultaneous hybridization with probes of SEQ ID Nº 1 and SEQ ID Nº 2 would lead to detection of *Streptococcus pneumoniae,* in a way that would allow distinguish it in a specific way from the other species of the *Streptococcus* genus. There is no explanation to be found either in the Melting Temperature, number of nucleotides, %GC (Table 1), or in the number of nucleotides by which these probes differ from the *Streptococcus pneumoniae* wild-type sequence (Table 2).

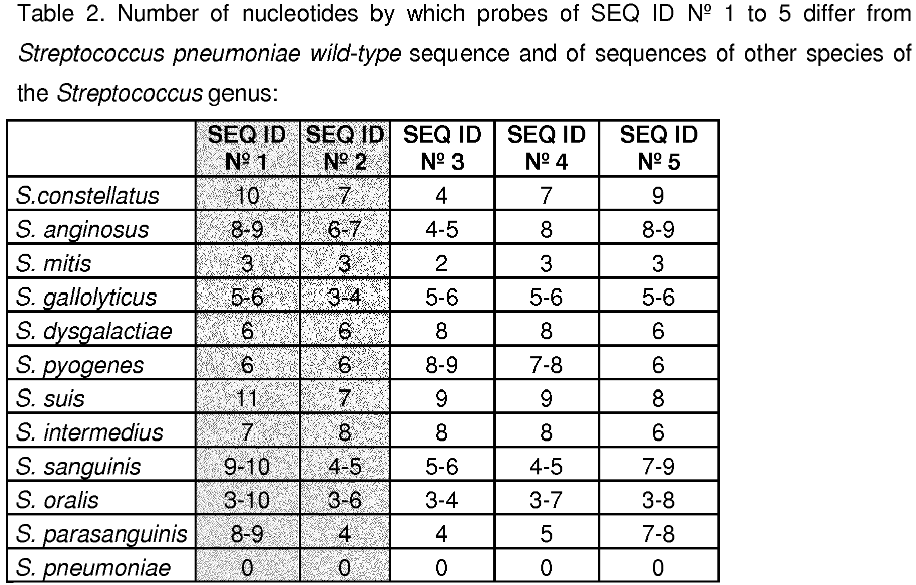

Different variants of the sequence of certain species have sometimes been detected. This is the reason why sometimes the number of nucleotides by which a certain probe differs from the sequence of a certain species of *Streptococcus* ranges between two values.

The inventors have thus surprisingly found that simultaneous incubation with probes of SEQ ID Nº 1 and SEQ ID Nº 2 allows detect and specifically distinguish *Streptococcus pneumoniae* from other species of the *Streptococcus* genus. Thus, simultaneous use of probes of SEQ ID Nº 1 and SEQ ID Nº 2 provides a solution to the problem of specific detection of *Streptococcus pneumoniae* in a sample. This is of great importance to distinguish *Streptococcus pneumoniae* from other microorganisms; In particular, from innocuous *Streptococcus* species constitutively present in the host and which do not cause disease, in particular, in the respiratory track. And also to detect presence of *Streptococcus pneumoniae* in blood, as a causative agent of sepsis.
There is nothing to be found in the state of the art, that might anticipate that probes of SEQ ID Nº 1 and SEQ ID Nº 2, when used simultaneously, would allow detect the presence of *Streptococcus pneumoniae* within a sample, and distinguish it from other species of the genera *Streptococcus.*

One of the aspects of the present invention thus corresponds to a method for the detection of *Streptococcus pneumoniae* in a test sample, the method comprising contacting the sample with probes of SEQ ID Nº 1 and SEQ ID Nº 2.
The probes may be provided in a microarray.

A second aspect of the present invention corresponds to a kit for the detection of *Streptococcus pneumoniae* in a test sample, wherein said kit comprises an array vessel or set of array vessels, each comprising a microarray wherein probes of SEQ ID Nº 1 and SEQ ID Nº 2 are provided.

Third and fourth aspects of the present invention respectively correspond to a set of probes comprising probes of SEQ ID Nº 1 and SEQ ID Nº 2, and to a microarray comprising this set of probes.

Further aspects of the present invention correspond to use of the method of claims 1 to 7, of the kit of claims 8 to 10, of the set of probes of claim 11 or of the microarray of claim 12, for detection of *Streptococcus pneumoniae,* and for diagnosis of an infection by *Streptococcus pneumoniae* in a patient. In particular, of a septic or respiratory infection.

### Brief description of the drawings.

Figure 1.

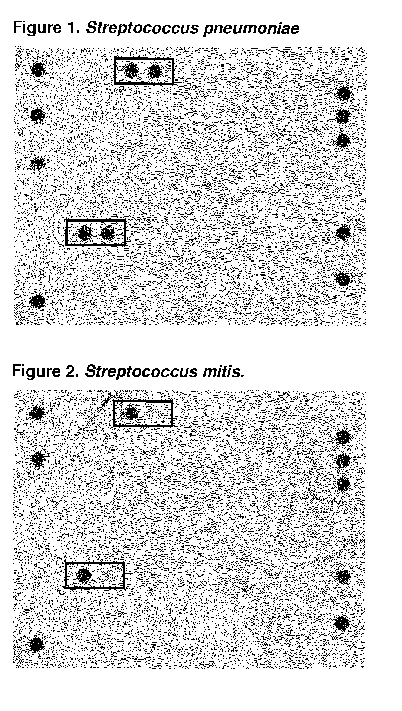

   Figure 1 shows visualization of hybridization of the amplification products of Example 1 of the present invention, previously denatured, with a microarray which comprises probes of SEQ ID Nº 1 and SEQ ID Nº 2 of the present invention.
   The "spots" surrounded by rectangles correspond to positions of the probes of SEQ ID Nº 1 and SEQ ID Nº 2. Of the two "spots" surrounded by each rectangle, the first one corresponds to SEQ ID Nº 1, and the second one to SEQ ID Nº 2. The "spots" outside the rectangles that display a signal, correspond to the Position Markers.
   The sample of Example 1, which contains *Streptococcus pneumoniae,* displays a positive signal and of equal intensity, in both the positions corresponding to probes of SEQ ID Nº 1 and SEQ ID Nº 2. This is due to the specific binding to both these probes of the amplified and denatured DNA of the sample.
Figure 2.
   Figure 2 displays visualization of hybridization of the amplification products of Example 2 of the present invention, previously denatured, with a microarray which comprises probes of SEQ ID Nº 1 and SEQ ID Nº 2 of the present invention.
   The "spots" surrounded by rectangles correspond to positions of the probes of SEQ ID Nº 1 and SEQ ID Nº 2. Of the two "spots" surrounded by each rectangle, the first one corresponds to SEQ ID Nº 1, and the second one to SEQ ID Nº 2. The "spots" outside the rectangles that display a signal, correspond to the Position Markers.
   The sample of Example 2, which contains *Streptococcus mitis,* displays a positive signal in the position corresponding to probe of SEQ ID Nº 1, an a signal of much lower intensity in that of probe of SEQ ID Nº 2. The signal that appears in position of SEQ ID Nº 2 has an intensity of less than 20% of the one that appears in position of SEQ ID Nº 1.
Figure 3.

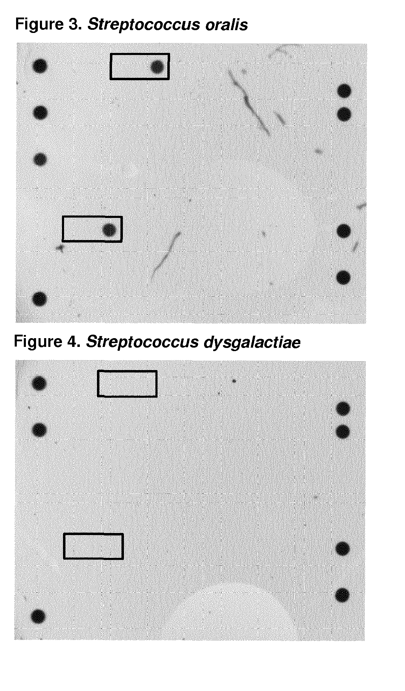

   Figure 3 shows visualization of hybridization of the amplification products of Example 3 of the present invention, previously denatured, with a microarray which comprises probes of SEQ ID Nº 1 and SEQ ID Nº 2 of the present invention.
   The "spots" surrounded by rectangles correspond to positions of the probes of SEQ ID Nº 1 and SEQ ID Nº 2. Of the two "spots" surrounded by each rectangle, the first one corresponds to SEQ ID Nº 1, and the second one to SEQ ID Nº 2. The "spots" outside the rectangles that display a signal, correspond to the Position Markers.
   The sample of Example 3, which contains *Streptococcus oralis,* displays a positive signal only in the position corresponding to probe of SEQ ID Nº 2, and does not display a signal in the position of the probe of SEQ ID Nº 1.
Figure 4.
   Figure 4 shows visualization of hybridization of the amplification products of Example 4 of the present invention, previously denatured, with a microarray which comprises probes of SEQ ID Nº 1 and SEQ ID Nº 2 of the present invention.
   The "spots" surrounded by rectangles correspond to positions of the probes of SEQ ID Nº 1 and SEQ ID Nº 2. Of the two "spots" surrounded by each rectangle, the first one corresponds to SEQ ID Nº 1, and the second one to SEQ ID Nº 2. The "spots" outside the rectangles that display a signal, correspond to the Position Markers.
   The sample of Example 4, which contains *Streptococcus dysgalactiae,* does not display any kind of signal in the positions of probes of SEQ ID Nº 1 nor SEQ ID Nº 2.

### Detailed description of the invention.

In the following passages, different aspects of the present invention are defined in more detail. Each aspect so defined may be combined with any other aspect or aspects unless clearly indicated to the contrary. In particular, any feature indicated as being preferred or especially advantageous may be combined with any other preferred or advantageous feature or features.

A first aspect of the present invention corresponds to a method for the detection of *Streptococcus pneumoniae* in a test sample, the method comprising contacting the sample with probes of SEQ ID Nº 1 and SEQ ID Nº 2.

A second aspect of the present invention relates to a kit for the detection of *Streptococcus pneumoniae* in a test sample, wherein said kit comprises an array vessel or set of array vessels, each comprising a microarray wherein probes of SEQ ID Nº 1 and SEQ ID Nº 2 are provided.

Third and fourth aspects of the present invention respectively correspond to a set of probes comprising probes of SEQ ID Nº 1 and SEQ ID Nº 2, and to a microarray comprising this set of probes.

Further aspects of the present invention relate to use of the method of claims 1 to 7, of the kit of claims 8 to 10, of the set of probes of claim 11 or of the microarray of claim 12, for detection of *Streptococcus pneumoniae,* and for diagnosis of an infection by *Streptococcus pneumoniae* in a patient. In particular, of a respiratory or septic infection.

### Definitions:

By the terms "probes of SEQ ID Nº 1 and SEQ ID Nº 2" it is understood both the nucleotide sequences depicted as SEQ ID Nº 1 and SEQ ID Nº 2, but also obvious equivalents to them, such as their complement sequences or sequences that have equivalent binding ability to that of SEQ ID Nº 1 and SEQ ID Nº 2 or that of their complement sequences (e.g. sequences that differ from sequences of SEQ ID Nº 1 and 2 or from their complement sequences by 1 mutation thereof).

"Primer", "Amplification Primer" or "PCR Amplification Primer" means "primer of a nucleic acid amplification reaction".
As "much lower extent" binding it is understood a binding level of 20% or lower than the one to which reference is made to.
Binding of the DNA to be tested and any probe, takes place through hybridization.

In preferred embodiments, the probes are provided in a microarray.
A microarray is a collection of microscopic oligonucleotide spots. A DNA microarray (also commonly known as gene chip, DNA chip, or biochip) is a collection of microscopic DNA spots attached to a solid surface. Probes are synthesized and then attached via surface engineering to a solid surface by a covalent bond to a chemical matrix (via epoxy-silane, amino-silane, lysine, polyacrylamide or others). Solid surfaces are known in the art and include microscopic beads as well as solid supports.
Thus, in particular, the probes of the present invention may be immobilized on a solid support. This solid support can be the bottom of an array vessel or a different solid support attached to the bottom of an array vessel. This means that the surface of the microarray may be the flat bottom of the array vessel. Alternatively, the surface of the microarray may be a solid support attached to the bottom of the array vessel.
The probes of the present invention may be included in an individual array vessel or in a set of array vessels.

Described herein are probes contained in a microarray, which may be placed on a slide or contained in a reaction vessel, which is then called an array vessel. Array vessels may have different formats of presentation, including individual array vessels, such as wells or tubes, or sets of array vessels arranged in strips of wells or tubes, or flat plates. Usually, plates consist of sets of strips of array vessels. Thus, the microarray of the present invention may be contained in an individual array vessel. Alternatively, two or more microarrays may be contained in a strip of vessels. In a preferred embodiment, the strip of vessels is constituted by 8 vessels. Further, three or more array vessels may be arranged in a set of strip of vessels. In another preferred embodiment, the set of strip of vessels is a microtiter plate. In yet another preferred embodiment, the microtiter plate is constituted by 96 array vessels.
The probes of the present invention can be obtained by different methods, such as chemical synthesis (e. g. by the conventional phosphotriester method); or genetic engineering techniques, for example by molecular cloning of recombinant plasmids in which corresponding nucleotide sequences have been inserted and can be latter obtained by digestion with nucleases.

The methods and kit of the present invention admit that the test sample may be amplified before being contacted with the probes of SEQ ID Nº 1 and SEQ ID Nº 2. Should this be the case, any components known by the skilled person to be necessary for nucleic acid amplification and, in particular, for PCR amplification, may also be present within the amplification mixture. Thereby, PCR Amplification Primers, appropriate nucleotide triphosphates, such as dATP, dCTP, dGTP, dTTP, and a suitable enzyme for polymerisation may also be present within the mixture of amplification reagents.
Any convenient enzyme for polymerisation may be used. In particular, HotStarTaq DNA Polymerase of QIAGEN Multiplex PCR Kit, performs particularly well. This DNA Polymerase is a modified form of Taq DNA polymerase which has no polymerase activity at ambient temperatures, and is activated by a 15-minute incubation at 95°C. Any other enzyme with these characteristics known in the state of the art, may also preferably be used.
Standard thermal cycling conditions may be used for carrying out amplification according to the present invention. Some specially preferred conditions are:

| **NUMBER OF CYCLES** | **TEMPERATURE** | **TIME** |
|---|---|---|
| 1 cycle | 95ºC | 15' |
| | 95ºC | 15-30" |
| 40-45 cycles | 55-59ºC | 15-60" |
| | 68-72ºC | 45-60" |
| 1 cycle | 68-72ºC | 10' |
| 1 cycle | 4ºC | Forever |

Some specially preferred PCR Amplification Primers are nucleic acid sequences which comprise any of the sequences of the group formed by SEQ ID Nº 6 and SEQ ID Nº 7 displayed in Table 3 below. Most preferably, PCR Amplification Primers consist of nucleic acid sequences SEQ ID Nº 6 and SEQ ID Nº 7.

**Table 3:**

| **SEQ ID N**º | **Primer name** | **5'-3'Sequence** |
|---|---|---|
| 6 | Stpn5 | TCTGAGTTGTCTMACAARCG |
| 7 | Stpn6r | GAATACATGCYGTCGCAACRGC |

| | | |
|---|---|---|
| M=A/C; R=A/G; Y=C/T | | |

It is not essential that amplification primers are those of SEQ ID Nº 6 and SEQ ID Nº 7. Other pairs of amplification primers that yield amplification products that include the region that hybridizes with probes of SEQ ID Nº 1 and SEQ ID Nº 2, may alternatively be used.
Present invention is compatible with the presence of amplification primers for other microorganisms within the same reaction mixture. Further, it is also compatible with inclusion of pairs of primers for amplification of, at least, one Internal Control, and one Extraction Control.
After the amplification process, preferably, the amplification product obtained is transformed into single-stranded DNA prior to hybridization with the corresponding probe. Most preferably single-stranded DNA is obtained through denaturation of the amplification product, most preferably, through heat-denaturation.

In cases where the sample to be tested is not amplified prior to incubation with probes, the nucleic acid present in the sample may be labelled by any DNA labelling method known in the art. Also, a label may be introduced in the DNA amplification product during amplification to allow further detection; in particular, a label that provides a signal that may be detected by colorimetric methods, by fluorescent methods, or by any labelling method known in the art. The label can be radioactive, chemiluminescent, luminescent and fluorescent agents. In a preferred aspect, the label that is used is biotin. However, any other kind of label known in the art may be used (eg. digoxigenin). In a preferred aspect, at least one of the primers used is labelled at the 5' end with biotin. Furthermore, labelling of amplified DNA may alternatively be achieved by adding modified nucleotides bearing a label (e.g. biotinylated or digoxigenin dUTP derivatives) to the PCR mixture. In certain embodiments, radioactive labels may be used, as well as fluorophores.

Alternative methods known to the skilled person, that may enable detection of the interaction between any amplification product and its corresponding probe, including methods that imply labelling of the probe, may be used.
The denatured amplification products can be incubated with probes immobilized in a microarray, either by themselves, or mixed with an hybridization solution comprising salts and detergents known by the person skilled in the art to favour interactions between DNA molecules.
Due to the labelling of the sample DNA, wherever sample molecules interact with probe molecules on the surface of the microarray, a reporter reagent binds the label and produces visible signals which may be detected by a detection device. The interacting probe and sample molecules are identified by the location of the signal on the surface of the microarray. In the particular case where sample amplification products are labelled with biotin, the reporter agent can be horseradish peroxidase covalently joined to streptavidin. The latter binds specifically to biotin, and the peroxidase triggers the precipitation of substrates like tetramethylbenzidine (TMB) or o-Dianisidine.
Any other detection method known in the state of the art, such as fluorescence, may be used for detection of the interaction between amplification products and corresponding probes.

Any other reaction that results in a precipitate on array elements, and that can be used to detect the interaction between target and probe molecules, may equally be used.

In a preferred embodiment, visualization of the interactions between target molecules and their corresponding specific or control probes, consists of the following steps:
- First, the image of the array is captured using an optical device;
- Then, the image is analysed;
- Finally, a report containing an interpretation of the result is provided.
Preferably, the image is analysed by means of appropriate software. Any device suitable for this processing can be used.

In a preferred embodiment, types of test samples that can be processed within the present invention are nasopharyngeal exudates, nasopharyngeal washes, sputum, bronchi-alveolar aspirates, and bronchi-alveolar washes. These would be the most suitable samples for diagnosis of respiratory infections. Further samples to be processed according to the present method and kit are blood and blood culture samples, which are most suitable in the case of patients with symptoms of sepsis. Any other body fluid, or tissue obtained from an individual may also be subjected to the method and kit of the present invention. The individual is human.

The test sample may equally be genetic material extracted from the original sample. Extraction of genetic material can be carried out both by automatic as well as by manual extraction techniques of the state of the art.
In a most preferred embodiment, the automatic extraction system is the NucliSENS easyMAG of BioMerieux (EP1694813), which uses magnetic particles in combination with BioMerieux's BOOM@ technology for universal isolation of total nucleic acid from a wide range of sample volumes and types. Other techniques of automatic extraction can equally be used.
The skilled person will also be aware of techniques and methods for manual processing of samples to extract nucleic acids; and any such suitable method may be used.
In a particular example, nucleic acids are extracted from the sample to be tested. Preferably, the initial volume is in the range of from 200 µl to 1 ml. After the extraction step, a precipitate is resuspended in a volume of from 25 µl to 80 µl of RNase-free water. In a particular embodiment, 5 µl of the 25 to 80 µl comprising the genetic material extracted from the test sample, are added to a vessel containing the amplification reactants, in a final volume of 50 µl.

The examples provided below merely illustrate the present invention and in no way limit the scope of the accompanying claims.

### Examples.

### Example 1:

A sample of blood culture positive for *Streptococcus pneumoniae* was obtained, and DNA was extracted from it by means of NucliSENS easyMAG kit of BioMerieux. The initial volume of blood culture that was used was of 1 ml, while the final volume after elution was of 80 µl.
Next, 5 µl of the eluted volume were taken and subjected to amplification with the following mixture of reagents:

| **Amplification Reagents** | **Final Concentration PCR tube (µM)** |
|---|---|
| Buffer Q 5x | 1X |
| 2X QIAGEN Multiplex PCR Master Mix | 1X |
| Primer SEQ ID Nº 6 | 0.8-2 |
| Primer SEQ ID Nº 7, labelled with Biotin | 0.8-2 |
| H₂O | Up to a final volumen of 45 µl |

| **NUMBER OF CYCLES** | **TEMPERATURE** | **TIME** |
|---|---|---|
| 1 cycle | 95ºC | 15' |
| | 95ºC | 15" |
| 40 cycles | 55ºC | 15" |
| | 68ºC | 45" |
| 1 cycle | 68ºC | 10' |
| 1 cycle | 4ºC | Forever |

The amplification products were denatured through incubation at 95°C for 10 minutes. After that, 5 µl of denatured PCR product were incubated, in the presence of a standard hybridization solution, with a microarray which comprises probes of SEQ ID Nº 1 and SEQ ID Nº 2 immobilized in known positions.
After a series of standard washing and blocking steps, an image was taken with the visualization of the final result that is shown in Figure 1.

### Example 2.

A sample of blood culture positive for *Streptococcus mitis* was obtained, and subjected to the same procedure described within Example 1 for *Streptococcus pneumoniae.* Visualization of the final result is displayed in Figure 2.

### Example 3.

A sample of blood culture positive for *Streptococcus oralis* was obtained, and subjected to the same procedure described within Example 1 for *Streptococcus pneumoniae.* Visualization of the final result is displayed in Figure 3.

### Example 4:

A sample of blood culture positive for *Streptococcus dysgalactiae* was obtained, and subjected to the same procedure described within Example 1 for *Streptococcus pneumoniae.* Visualization of the final result is displayed in Figure 4.

## Claims

1. A method for the detection of *Streptococcus pneumoniae* in a test sample, the method comprising contacting the sample with probes of SEQ ID Nº 1 and SEQ ID Nº 2.

2. The method of claim 1 wherein prior to the contact with the probes, the sample is subjected to PCR amplification.

3. The method of claim 2 wherein two nucleic acid sequences respectively comprising SEQ ID Nº 6 and SEQ ID Nº 7, are used as PCR amplification primers.

4. The method of claims 1 to 3 wherein single-stranded oligo- or polynucleotides are obtained from the sample or from any PCR amplification products obtained from it, prior to contacting with the probes of SEQ ID Nº 1 and SEQ ID Nº 2.

5. The method of claim 4 wherein single-stranded oligo- or polynucleotides are obtained by denaturing any double-stranded oligo- or polynucleotides present in the sample or in the amplification products obtained from it.

6. The method of claims 1 to 5 wherein the probes of SEQ ID Nº 1 and SEQ ID Nº 2 are provided in a microarray.

7. The method of claims 1 to 6 wherein the probes of SEQ ID Nº 1 and SEQ ID Nº 2 are included in an individual array vessel, or in a set of array vessels.

8. A kit for the detection of *Streptococcus pneumoniae* in a test sample, wherein said kit comprises an array vessel or set of array vessels, each comprising a microarray wherein probes of SEQ ID Nº 1 and SEQ ID Nº 2 are provided.

9. The kit of claim 8, wherein said kit further comprises:
- i) reagents for use in visualising hybridisation of nucleic acids to the probes of the microarray, and
- ii) an amplification mixture with PCR amplification primers.

10. The kit of claim 9 wherein the amplification mixture comprises two nucleic acid sequences, respectively comprising SEQ ID Nº 6 and SEQ ID Nº 7, as PCR amplification primers.

11. A set of probes comprising probes of SEQ ID Nº 1 and SEQ ID Nº 2.

12. A microarray comprising the set of probes as defined in claim 11.

13. Use of the method of claims 1 to 7, of the kit of claims 8 to 10, of the set of probes of claim 11 or of the microarray of claim 12, for detection of *Streptococcus pneumoniae.*

14. Use of the method of claims 1 to 7, of the kit of claims 8 to 10, of the set of probes of claim 11 or of the microarray of claim 12, for diagnosis of an infection by *Streptococcus pneumoniae* in a patient.

15. Use of claim 14 wherein the infection is a septic or respiratory infection.


SEQUENCE LISTING

<110> GENOMICA S.A.U.

<120> Detection of Streptococcus pneumoniae.

<130> Streptococcus EP2013

<160> 7

<170> PatentIn version 3.3

<210> 1
<211> 24
<212> DNA
<213> Artificial Sequence
<220>
<223> Probe for specific detection of Streptococcus pneumoniae

<400> 1

gatggaacct ttgctgagaa gatt 24

<210> 2
<211> 23
<212> DNA
<213> Artificial Sequence
<220>
<223> Probe for specific detection of Streptococcus pneumoniae

<400> 2

tatactgtag ctcaggctaa ctc 23

<210> 3
<211> 23
<212> DNA
<213> Artificial Sequence
<220>
<223> Probe that does not allow specific detection of Streptococcus
pneumoniae

<400> 3

aaacaggtgt tgtcacgaac gaa 23

<210> 4
<211> 22
<212> DNA
<213> Artificial Sequence
<220>
<223> Probe that does not allow specific detection of Streptococcus
pneumoniae

<400> 4

gctcaggctaactctcgtctga                                                22

<210> 5
<211> 20
<212> DNA
<213> Artificial Sequence
<220>
<223> Probe that does not allow specific detection of Streptococcus
pneumoniae

<400> 5
gaacctttgc tgagaagatt                                                  20

<210> 6
<211> 20
<212> DNA
<213> Artificial Sequence
<220>
<223> Primer for PCR amplification of Streptococcus pneumoniae

<400> 6
tctgagttgt ctmacaarcg                                                  20
M=A/C; R=A/G; Y=C/T

<210> 7
<211> 22
<212> DNA
<213> Artificial Sequence
<220>
<223> Primer for PCR amplification of Streptococcus pneumoniae

<400> 7
gaatacatgc ygtcgcaacr gc                                               22
M=A/C; R=A/G; Y=C/T